**Europäisches Patentamt**

**European Patent Office**

**Office européen des brevets**

(19)

(11) Veröffentlichungsnummer : **0 044 877**
**B1**

(12) **EUROPÄISCHE PATENTSCHRIFT**

(45) Veröffentlichungstag der Patentschrift :
**13.03.85**

(21) Anmeldenummer : **80104410.8**

(22) Anmeldetag : **26.07.80**

(51) Int. Cl.⁴ : **A 61 B 5/10, A 61 B 17/42**

(54) Vorrichtung zum Bestimmen der Innenmasse des Cavum uteri.

(43) Veröffentlichungstag der Anmeldung :
**03.02.82 Patentblatt 82/05**

(45) Bekanntmachung des Hinweises auf die Patenterteilung : **13.03.85 Patentblatt 85/11**

(84) Benannte Vertragsstaaten :
**DE FR GB NL**

(56) Entgegenhaltungen :
**CH-B- 120 378**
**DE-A- 2 756 427**
**DE-C- 78 155**
**DE-C- 597 749**
**US-A- 4 141 345**

(73) Patentinhaber : **Kurz, Karl-Heinz, Dr. med.**
**Rheinbabenstrasse 5**
**D-4000 Düsseldorf (DE)**

**Gutiérrez, Javier, Dipl.-Ing.**
**Blumenstrasse 5**
**D-4005 Meerbusch 1 (DE)**

(72) Erfinder : **Kurz, Karl-Heinz, Dr. med.**
**Rheinbabenstrasse 5**
**D-4000 Düsseldorf (DE)**
Erfinder : **Gutiérrez, Javier, Dipl.-Ing.**
**Blumenstrasse 5**
**D-4005 Meerbusch 1 (DE)**

(74) Vertreter : **Patentanwälte, Dipl.-Ing. Klaus Westphal**
**Dr. rer. nat. Bernd Mussgnug Dr. rer. nat. Otto**
**Buchner**
**Waldstrasse 33**
**D-7730 Villingen-Schwenningen (DE)**

**Beschreibung**

Die Erfindung betrifft eine Vorrichtung zum Bestimmen der Innenmaße des Cavum uteri, bestehend aus einer in einer Hülse verschiebbaren Sonde, an deren Spitze bis zur Berührung mit der uteruswand aufspreizbare Fühler angeordnet sind, und bei der die Größe der Aufspreizung durch ein mechanisches Mittel zu einer am Fuß der Sonde befindlichen Anzeigevorrichtung übertragen wird.

Vorrichtungen dieser Art sind beispielsweise aus der DE-OS 27 56 427 bekannt. Eine möglichst exakte Bestimmung der Innenmaß des Cavum uteri, und zwar sowohl der seitlichen Ausdehnung als auch der Länge, ist unerläßlich vor dem Einsetzen intrauteriner Antikonzeptiva, bzw. Pessare. Die Pessare müssen den inneren Abmessungen des Cavum uteri angepaßt werden, andernfalls können sie unwirksam sein oder Komplikationen der verschiedensten Art verursachen. Davon abgesehen gibt es noch eine Vielzahl weiterer Gründe, die eine genaue Bestimmung der Innenmaße des Cavum uteri wünschenswert machen.

Mit der Vorrichtung gemäß der DE-OS 27 56 427 läßt sich diese Bestimmung zufriedenstellend bewerkstelligen. Sofern jedoch nicht nur der Abstand zwischen den beiden internen Tubenostien im Bereich des Fundusdaches ermittelt werden soll, sondern die tatsächliche Ausdehnung des gesamten Hohlraumes, ist eine Vielzahl von Einzelmessungen erforderlich, was zeitraubend ist. Darüber hinaus läßt sich ein Abbild des Hohlraumes nur unter der Voraussetzung ermitteln, daß er symmetrisch ist. Ferner hat sich gezeigt, daß bei der bekannten Vorrichtung der zur Übertragung des Maßes der Aufspreizung der Fühler zur Anzeigevorrichtung verwendete Faden nicht unproblematisch ist hinsichtlich einer ausreichend sterilen Handhabung.

Der Erfindung liegt daher die Aufgabe zugrunde, eine Vorrichtung zum Bestimmen der Innenmaße des Cavum uteri zu schaffen, mit der mittels einer einzigen Messung die seitlichen Konturen des Hohlraumes ermittelt werden können. In ihrer Weiterbildung soll es die Erfindung auch ermögliche, unsymmetrische Verläufe der seitlichen Konturen des Hohlraumes zu bestimmen. Darüber hinaus soll eine sterile Handhabung der Vorrichtung gewährleistet sein, ohne daß es dazu eines zusätzlichen Aufwandes bedarf.

Ausgehend von dem im Oberbegriff des Anspruches berücksichtigten Stand der Technik ist diese Aufgabe erfindungsgemäß gelöst mit den im kennzeichnenden Teil des Anspruches 1 angegebenen Maßnahmen.

Vorteilhafte Weiterbildungen der Erfindung sind in den Unteransprüchen angegeben.

Die erfindungsgemäße Vorrichtung enthält nur leicht steril zu haltende mechanische Teile. Insbesondere die Ausbildungsform gemäß Anspruch 5 ermöglicht eine sichere sterile Handhabung.

Bei der erfindungsgemäßen Vorrichtung wird die jeweilige Aufspreizung der Fühler in eine entsprechende Aufspreizung der Arme der Anzeigevorrichtung transformiert, so daß mit einem einzigen Durchfahren der Meßvorrichtung in Längsrichtung der gesamte Verlauf der seitlichen Konturen des Cavum uteri nach außen übertragen wird. Besonders vorteilhaft ist hierbei die Ausführungsform gemäß Anspruch 4, mit der eine grafische Aufzeichnung dieses Verlaufes erhalten werden kann.

Die Abwandlung der erfindungsgemäßen Vorrichtung gemäß Anspruch 2 ermöglicht es, auch einen unsymmetrischen Verlauf der seitlichen Konturen des Cavum uteri zu bestimmen.

Zwei Ausführungsbeispiele der Erfindung sollen anhand der beigefügten Zeichnungen erläutert werden.

Es zeigen :

Figur 1 eine erfindungsgemäße Vorrichtung in perspektivischer Darstellung,

Figur 2 eine seitliche Ansicht der Fig. 1 im Schnitt,

Figur 3 eine Draufsicht der Fig. 1 im Schnitt,

Figur 4 das Gelenkparallelogramm der Fühler von Fig. 1 im Detail,

Figur 5 das Gelenkparallelogramm der Anzeigevorrichtung von Fig. 1 im Detail,

Figur 6 die mit der Anzeigevorrichtung von Fig. 1 erhaltene grafische Darstellung und

Figur 7 eine Ausführungsform der Erfindung, mit welcher auch ein unsymmetrischer Verlauf der seitlichen Konturen des Cavum uteri ermittelt werden kann.

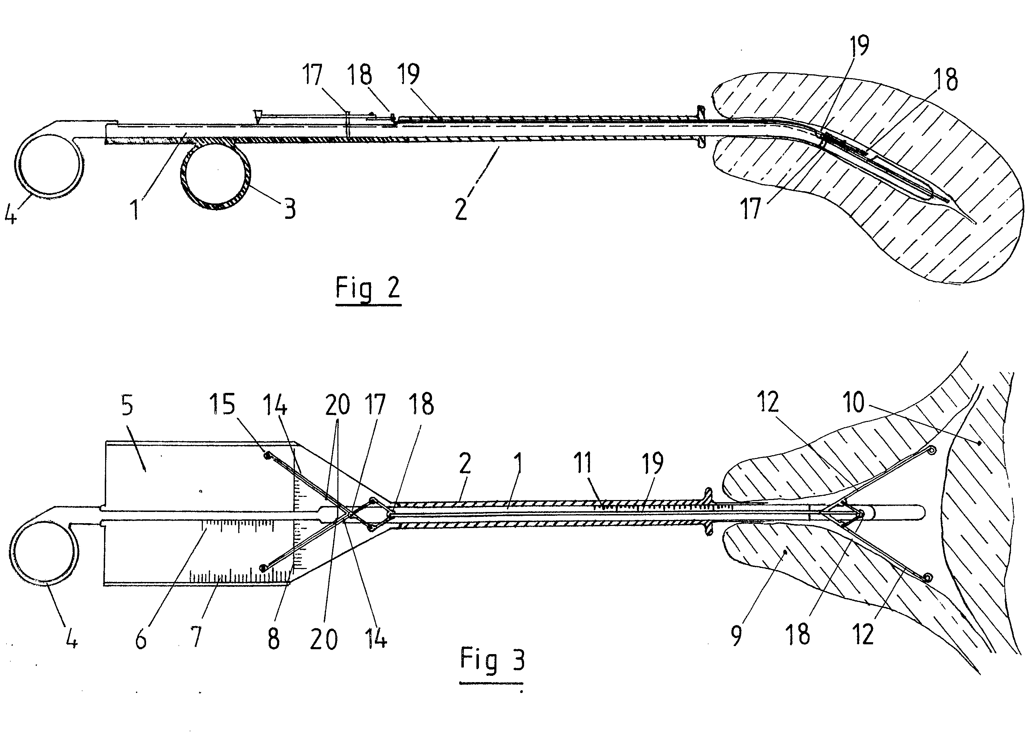

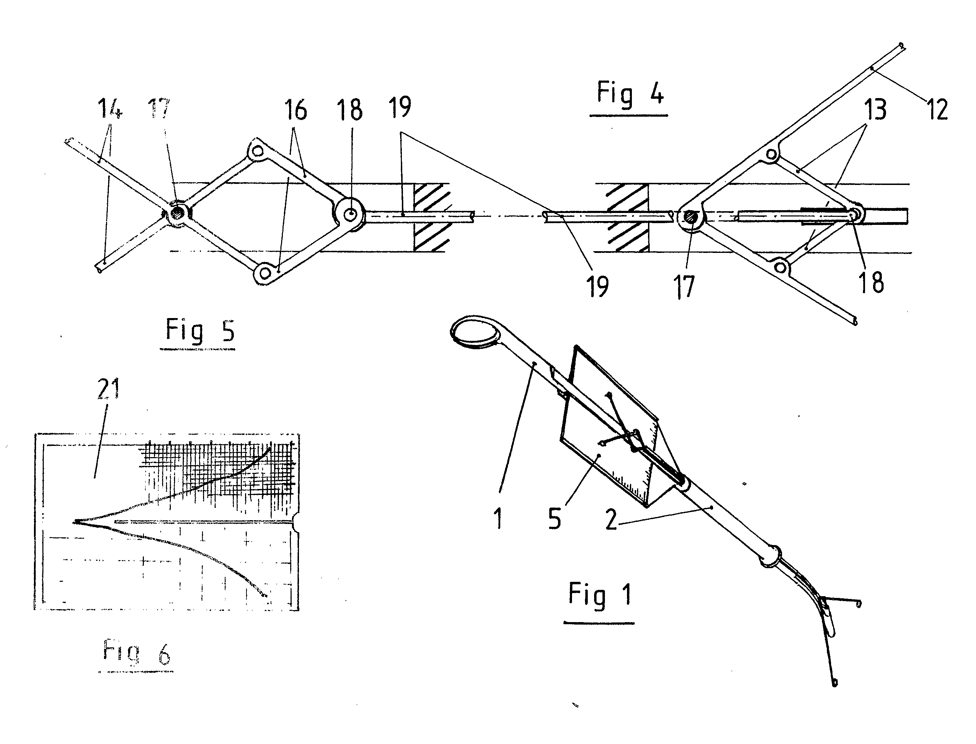

Die in den Fig. 1 bis 6 dargestellte Vorrichtung besteht im wesentlichen aus der Sonde 1, die in einer Hülse 2 verschiebbar angeordnet ist. Die Verschiebung wird durch die Grifflöcher 3 und 4 erleichtert.

Die Hülse 2 ist an einem Ende, nämlich im Bereich der Anzeigevorrichtung als Schreibplatte 5 ausgebildet, auf der Skalen 6, 7 und 8 in cm-Einteilung aufgebracht sind.

Zur Bestimmung der Länge des Cavum uteri wird die Hülse 2 am Muttermund 9 angesetzt und die Sonde 1 bis zum Fundusdach 10 eingeführt. Die Länge des Cavum uteri kann dann an der Skala 6 abgelesen werden. Falls gewünscht, kann dies auch an der Skala 11 erfolgen, sofern auf der Sonde 1 im Bereich der Skala 11 ein Zeiger befestigt ist, der durch einen Schlitz in der Hülse 2 auf der Skala 11 verschiebbar ist.

Zur Bestimmung der seitlichen Ausdehnung des Cavum uteri dienen die Fühler 12. Die Fühler 12 stellen die verlängerten Arme eines nach dem Prinzip des Pantografen ausgebildeten Gelenkparallelogramms dar. Mit 13 sind die beiden weiteren Arme des Gelenkparallelogramms bezeichnet.

Der Pantograf ist ein Gerät, um Zeichnungen, Pläne, Diagramme und dergl. zu vergrößern oder zu verkleinern. Er besteht aus einem Gelenkparallelogramm, dessen einer Punkt (Pol) festgehalten wird. Die zu vergrößernde Zeichnung wird mit einem Punkt eines Armes des Gelenkparallelogramms abgefahren. Ein Punkt auf einem anderen verlängerten Arm des Gelenkparallelogramms liefert dann die gleiche Zeichnung im vergrößerten Maßstab. Verkleinerungen sind sinngemäß ebenfalls möglich.

Die Erfindung macht von diesem Prinzip Gebrauch, jedoch mit dem Unterschied, daß keine Vergrößerung oder Verkleinerung der Vorlage, d. h. der Ausdehnung des Cavum uteri angestrebt wird, obwohl dies möglich wäre. Es wird vielmehr der mit dem pantografenähnlichen Gelenkparallelogramms aus den Fühlern 12 und den Armen 13 gewonnene Verlauf der seitlichen Konturen des Cavum uteri in gleichem Maßstab auf die Anzeigevorrichtung übertragen, indem diese ebenfalls als pantografenähnliches Gelenkparallelogramm ausgebildet ist.

Die Anzeigevorrichtung besteht demnach aus den Armen 14, die die gleiche Länge wie die Fühler 12 besitzen, und an deren Enden Markierungsstifte 15 angebracht sind. Die beiden anderen Arme des Gelenkparallelogramms der Anzeigevorrichtung sind mit 16 bezeichnet.

Jedes Gelenkparallelogramm ist mit einem Gelenk, dem Pol, auf der Sonde 1 befestigt. Diese Pole sind einheitlich mit 17 bezeichnet. Die den Polen 17 gegenüberliegenden Gelenke der Gelenkparallelogramme sind einheitlich mit 18 bezeichnet. Zwecks Übertragung der Aufspreizung der Fühler 12 auf die Arme 14 der Anzeigevorrichtung sind gemäß der Erfindung die Gelenke 18 durch eine Übertragungsstange 19 miteinanderverbunden. Es ist ohne weiteres ersichtlich, daß die Funktion der Pole 17 mit der der Gelenke 18 austauschbar ist.

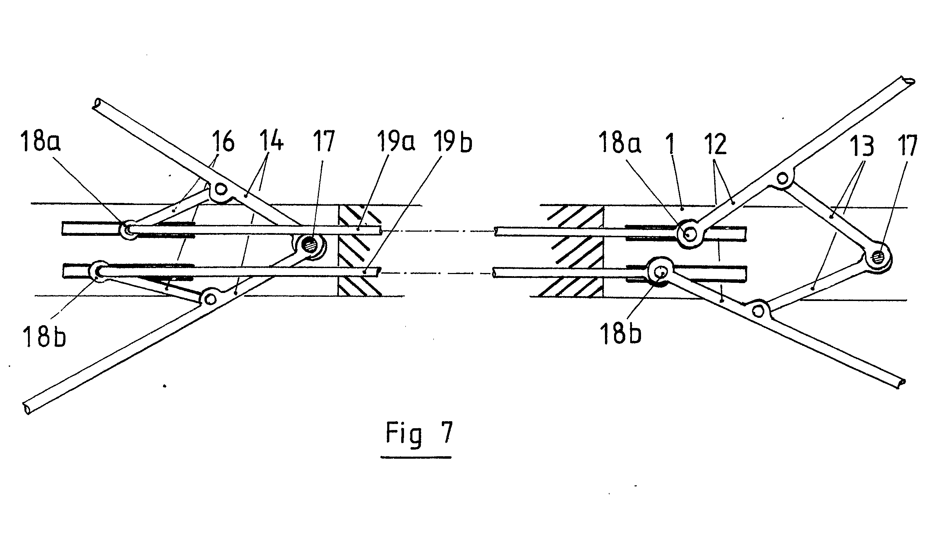

An die Arme 14 der Anzeigevorrichtung greift eine Feder 20 an, die sie auseinanderzuspreizen sucht. Da diese Aufspreizung mittels der Übertragungsstange 19 auch den Fühlern 12 aufgeprägt wird, haben diese die Tendenz, sich ebenfalls aufzuspreizen. Die Fühler 12 legen sich daher an die seitlichen Wände des Cavum uteri an. Je nachdem, wie tief die Sonde 1 eingefahren ist, spreizen sich die Fühler mehr oder weniger weit auf. Entsprechend verläuft die Aufspreizung der Arme 14 oder Anzeigevorrichtung. Mittels der Skalen 7 und 8 kann daher das Cavum uteri punktförmig ausgemessen werden. Vorzugsweise wird jedoch auf die Schreibplatte 5 ein mit Skaleneinteilung versehenes Papier 21 aufgespannt. Auf diesem Papier zeichnen die Markierungsstifte 15 in einem Zug die seitlichen Konturen des Cavum uteri. Dies ist in Fig. 6 dargestellt.

Die Fig. 7 zeigt in prinzipieller Darstellung eine Ausführungsform der erfindungsgemäßen Vorrichtung, die es gestattet, auch einen unsymmetrischen Verlauf der seitlichen Konturen des Cavum uteri auszumessen. Die Numerierung der Teile aus den Fig. 1 bis 6 wurde beibehalten. Gegensatz zur Ausführungsform gemäß den Fig. 1 bis 6 ist hierbei die Funktion der Pole 17 und der Gelenke 18 vertauscht, auf welche Möglichkeit bereits oben hingewiesen wurde. Für die Eignung der Vorrichtung zur Ausmessung unsymmetrischer seitlicher Konturen des Cavum uteri ist dies aber unerheblich. Entscheidend ist vielmehr die Aufteilung der Gelenke 18 in separate Gelenke 18a und 18b, die durch separate Übertragungsstangen 19a und 19b miteinander verbunden sind. Jeder Fühler 12 spreizt sich daher unabhängig vom anderen Fühler auf, soweit es die jeweilige seitliche Kontur des Cavum uteri gestattet. Entsprechend erfolgt die Aufspreizung der Arme 14 der Anzeigevorrichtung.

## Ansprüche

1. Vorrichtung zum Bestimmen der Innenmaße des Cavum uteri, bestehend aus einer in einer Hülse verschiebbaren Sonde, an deren Spitze bis zur Berührung mit der Uteruswand aufspreizbare Fühler angeordnet sind, und bei der die Größe der Aufspreizung durch ein mechanisches Mittel zu einer am Fuß der Sonde befindlichen Anzeigevorrichtung übertragen wird, gekennzeichnet durch folgende Merkmale :

a) die Fühler (12) sind die verlängerten Arme eines nach dem Prinzip des Pantografen (Storchschnabel) ausgebildeten Gelenkparallelogramms ;

b) die Anzeigevorrichtung besteht aus den verlängerten Armen (14) eines nach dem Prinzip des Pantografen (Storchschnabel) ausgebildeten Gelenkparallelogramms ;

c) die Pole (feste Gelenke) (17) der Gelenkparallelogramme sind auf der verschiebbaren Sonde (1) befestigt ;

d) die den Polen gegenüberliegenden beweglichen Gelenke (18) der Gelenkparallelogramme sind durch eine in der Hülze (2) geführte Übertragungsstange (19) so miteinander verbunden, daß ein Aufspreizen der Fühler ein entsprechendes Aufspreizen der Arme (14) der Anzeigevorrichtung bewirkt.

2. Abwandlung der Vorrichtung nach Anspruch 1, gekennzeichnet, durch folgende Merkmale :

e) die beweglichen Gelenke (Merkmal d)) der Gelenkparallelogramme sind in jeweils zwei separate Gelenke (18a, 18b) aufgeteilt, so daß die separaten Gelenke die vier Endpunkte von jeweils zwei Armen der Gelenkparallelogramme darstellen ;

f) die jeweils zwei separaten Gelenke des einen Gelenkparallelogramms sind durch zwei in der Hülse (2) geführte parallele Übertragungsstangen (19a, 19b) mit den entsprechenden separaten Gelenken des anderen Gelenkparallelogramms verbunden.

3. Vorrichtung nach Anspruch 1 oder 2, dadurch gekennzeichnet, daß die Arme (14) der Anzeigevorrichtung durch eine Feder (20) auseinandergespreizt werden.

4. Vorrichtung nach einem der Ansprüche 1 bis 3, gekennzeichnet, durch folgende Merkmale :

g) an den Endpunkten der Arme (14) der Anzeigevorrichtungen sind Markierungsstifte (15) angeordnet ;

h) die Hülse ist im Bereich der Anzeigevorrichtung als Schreibplatte (5) ausgebildet, auf der ein mit Skaleneinteilungen (6, 7, 8) versehenes Papier (21) zur Aufzeichnung der Konturen des Cavum uteri aufgespannt werden kann.

5. Vorrichtung nach einem der Ansprüche 1 bis 4, gekennzeichnet durch eine derartige Gestaltung der Sonde mit Gelenkparallelogrammen, daß sie aus der Hülse herausziehbar ist.

## Claims

1. A device for determining the internal dimensions of the cavum uteri, consisting of a probe which is displaceable in a sleeve and at the tip of which feelers are arranged which can be extended until they touch the wall of the uterus, and in which the magnitude of the expansion is transmitted by mechanical means to an indicator device located at the foot of the probe, characterised by the following features :

a) the feelers (12) are the extended arms of a link parallelogram formed in accordance with the pantograph principle ;

b) the indicator device consists of extended arms (14) of a link parallelogram formed in accordance with the pantograph principle ;

c) the poles (fixed joints) (17) of the link parallelograms are fixed to the displaceable probe (1) ;

d) the movable joints (18), located opposite the poles, of the link parallelograms are mutually connected by a transmission rod (19) guided in the sleeve (2), in such a way that an expansion of the feelers effects a corresponding expansion of the arms (14) of the indicator device.

2. Modification of the device according to Claim 1, characterised by the following features ;

e) the movable joints (feature d) of the link parallelograms are each divided into two separate joints (18a, 18b), so that the separate joints represent, respectively, the four end points of two arms of the link parallelograms ;

f) the two separate joints of one link parallelogram are respectively connected by two parallel transmission rods (19a, 19b), guided in the sleeve (2), to the corresponding separate joints of the other link parallelogram.

3. A device according to Claim 1 or 2, characterised in that the arms (14) of the indicator device are spread apart by a spring (20).

4. Device according to any one of Claims 1 to 3, characterised by the following features :

g) marking pins (15) are arranged at the end points of the arms (14) of the indicator device ;

h) in the region of the indicator device, the sleeve is designed as a writing plate (5), on which a paper sheet (21) provided with scale divisions (6, 7, 8) can be fitted for recording the contours of the cavum uteri.

5. A device according to any one of Claims 1 to 4, characterised by a design of the probe with link parallelograms such that it is withdrawable from the sleeve.

## Revendications

1. Dispositif pour déterminer les dimensions intérieures de la cavité utérine (cavum uteri), comportant une sonde déplaçable dans un manchon, au bout de laquelle sont disposés des palpeurs capables d'un écartement jusqu'à venir au contact de la paroi utérine et dont l'écartement est transmis, par voie mécanique, à un dispositif indicateur disposé au pied de la sonde, caractérisé en ce que :

a) les palpeurs (12) sont les bras prolongés d'un parallélogramme articulé construit selon le principe du pantographe ;

b) le dispositif indicateur est constitué par les bras prolongés (14) d'un parallélogramme articulé construit selon le principe du pantographe ;

c) les pôles (articulations fixes) (17) des parallélogrammes articulés sont fixés sur la sonde déplaçable (1) ;

d) les articulations mobiles (18) opposées aux pôles des parallélogrammes articulés sont reliées entre elles par une tige de transmission (19) guidée dans le manchon (2) de telle manière qu'un écartement des palpeurs provoque un écartement correspondant des bras (14) du dispositif indicateur.

2. Modification du dispositif selon la revendication 1, caractérisée en ce que :

e) les articulations mobiles (caractéristique d) des parallélogrammes articulés sont subdivisées respectivement en deux articulations séparées (18a, 18b) de sorte que les articulations séparées constituent les quatre points extrêmes de deux paires de bras des parallélogrammes articulés ;

f) les deux articulations séparées de l'un des parallélogrammes articulés sont reliées aux articulations séparées correspondantes de l'autre parallélogramme articulé par deux tiges de transmission (19a, 19b) parallèles guidées dans le manchon (2).

3. Dispositif selon l'une des revendications 1 ou 2, caractérisé en ce que les bras (14) du dispositif indicateur sont écartés par un ressort (20).

4. Dispositif selon l'une quelconque des revendications 1 à 3, caractérisé en ce que :

g) des stylets inscripteurs (15) sont disposés aux extrémités des bras (14) des dispositifs indicateurs ;

h) dans la zone du dispositif indicateur, le manchon se déploie en forme de tablette à écrire (5) sur laquelle on peut fixer une feuille de papier (21) pourvue d'échelles graduées (6, 7, 8) pour enregistrer les contours de la cavité utérine.

5. Dispositif selon l'une quelconque des revendications 1 à 4, caractérisé par une configuration de la sonde avec parallélogrammes articulés telle qu'elle puisse être retirée du manchon.

Fig 2

Fig 3

0 044 877

Fig 4

14  17      16  18  19                          13  12

19      17                          18

Fig 5

21

Fig 6

1   5   2

Fig 1

0 044 877

Fig 7